# EUROPEAN PATENT SPECIFICATION

(11) **EP 1 262 173 B1**
(45) Date of publication and mention of the grant of the patent: **11.08.2004**
(21) Application number: 02253264.2
(22) Date of filing: 09.05.2002
(51) Int. Cl.: A61K 9/08, A61K 47/04

(54) **Oral intake solution comprising reduced water**
Zubereitung enthaltend reduziertes Wasser
Composition comprenant eau réduite

(30) Priority: 17.05.2001 JP 2001147949
(43) Date of publication of application: 04.12.2002
(73) Proprietor: Nihon Trim Co. Limited, Osaka-Shi, Osaka (JP)
(72) Inventor: Kataoka, Masumi, Osaka-shi, Osaka (JP); Gondo, Yumiko, Akashi-shi, Hyogo (JP); Morisawa, Shinkatsu, Kyoto-shi, Kyoto (JP)
(74) Representative: Matthews, Heather Clare

(56) References cited:
- EP-A- 1 081 096

## Description

### BACKGROUND OF THE INVENTION

### Field of the Invention

The present invention relates to a method for reducing a bitter taste and an oral intake solution with reduced bitterness.

### Description of the Background Art.

Vitamin B compounds are useful for mammals. Its oral administration, however, is difficult due to the characteristic bitter taste. Conventional vitamin B-containing oral intake solutions have sucrose, honey or the like blended thereto, to reduce the bitterness to make it easier to take orally. Still, they cannot eliminate the bitter taste completely.

Various techniques have been developed to reduce such bitterness. Japanese Patent Laying-Open No. 5-4921 discloses a vitamin B-containing oral solution agent blended with amino acids and apple flavor. Japanese Patent Laying-Open No. 5-255126 discloses a composition prepared by combining essential oil or component thereof. Japanese Patent Laying-Open No. 9-328429 discloses blending of licorice extract and nonionic surfactant to the vitamin B₁ derivative. Japanese Patent Laying-Open No. 11-209266 discloses an oral intake solution containing thiamine or its salt, added with a ginger group flavor.

However, there are unfulfilled demands for novel techniques that can reduce bitterness of a water-soluble component like vitamin B compound.

### SUMMARY OF THE INVENTION

An object of the present invention is to provide a novel method for reducing a bitter taste and an oral intake solution with reduced bitterness.

To achieve such an object, the inventors have found through various studies that electrolytic reduced water (i.e., reduced water obtained by electrolysis) is effective to reduce bitterness of vitamin B compound, and have reached the present invention.

More specifically, a method for reducing bitterness according to an aspect of the present invention is characterized in that a vitamin B compound as a water-soluble component giving a bitter taste is dissolved in reduced water, having an oxidation potential of less than 0mV.

The reduced water is preferably electrolytic reduced water.

An oral intake solution according to another aspect of the present invention contains a vitamin B compound as a water-soluble component giving a bitter taste and reduced water, having an oxydation potential of less than 0mV. The reduced water is preferably electrolytic reduced water. Further, the oral intake solution preferably has a pH value in a range from 2 to 7.

A method for producing an oral intake solution according to a further aspect of the present invention is characterized in that a vitamin B compound as a water-soluble component giving a bitter taste is dissolved in reduced water.

The foregoing and other objects, features, aspects and advantages of the present invention will become more apparent from the following detailed description of the present invention.

### DESCRIPTION OF THE PREFERRED EMBODIMENTS

The water-soluble component giving a bitter taste to be used in the present invention is the vitamin B compound that dissolves into water and leaves bitterness in oral cavities after administered. Herein, the vitamin B compound refers to compounds belonging to the vitamin B group, and their derivatives and pharmaceutically acceptable salts. Specifically, the vitamin B compound includes: thiamine; thiamine derivatives (e.g., prosultiamine, fursultiamine, octotiamine, allithiamine, thiamine disulfide, O-benzoyl thiamine disulfide, thiamine mono-phosphate disulfide, O,S-dibenzoyl thiamine, S-benzoyl thiamine, benfotiamine, dicethiamine, diclocarbothiamine) and their pharmaceutically acceptable salts (e.g., acid-added salts including: hydrochloride such as thiamine hydrochloride, nitrate such as thiamine nitrate, phosphate, and sulfate); riboflavin; riboflavin derivatives (e.g., riboflavin butyrate, sodium riboflavin phosphate); pyridoxine; pharmaceutically acceptable salts of pyridoxine (e.g., pyridoxine hydrochloride); pyridoxine derivatives (e.g., pyridoxal phosphate, pyridoxamine phosphate); and, as vitamin B₁₂, cyanocobalamin, hydroxocobalamin, hydroxocobalamin acetate, methylcobalamin. These may be used alone, or two or more of them may be employed together.

The reduced water for use in the present invention refers to water having strong reduction power compared to general tap water and mineral water. It has an oxidation potential of less than 0 mV preferably not greater than -100 mV, and more preferably not greater than -500 mV. The reduced water may be produced by subjecting tap water to electrolysis, magnetizing process, electronic process, ultrasonic process, crystal or mineral process (e.g., tourmaline, biotite monzonite spotted stone (bakuhanseki), quartz diorite porphyrite (iouseki)), or other process. Alternatively, the reduced water present in nature may be employed. In the present invention, the reduced water is preferably produced by subjecting electrolyte-containing water to electrolysis; the water thus produced is suitably used for the invention. Herein, the reduced water obtained by electrolysis is called "electrolytic reduced water".

More specifically, the electrolytic reduced water may be produced by subjecting purified water containing 0.001-0.01% sodium chloride to strong decomposition employing a commercially available, running-water type electrolyzer (TI-8000 model, manufactured by NIHON TRIM CO., LTD.)

The reduced water contains dissolved hydrogen (molecular hydrogen). From the standpoint of effectively masking the bitter taste, the content of the dissolved hydrogen has its lower limit of preferably 400 ppb, more preferably 880 ppb, and its upper limit of preferably 1100 ppb, more preferably 1060 ppb. The concentration of the dissolved hydrogen may be measured using a commercially available measurement device (e.g., DHDI-1 model, manufactured by DKK-TOA Corporation).

According to the method of the present invention, a bitter taste of the vitamin B compound as a water-soluble component giving bitterness can be lessened by dissolving the component into the reduced water. For the dissolution of the component, any known method may be employed. Although the component may be blended in any amount within a range ensuring reduction of the bitterness, the component is blended preferably 0.001-0.05 parts by weight, more preferably 0.002-0.04 parts by weight, with respect to 1 part by weight of the electrolytic reduced water.

The oral intake solution of the present invention can be produced according to the inventive bitterness-reducing method. The inventive solution has a pH value preferably in a range from 2 to 7, more preferably in a range from 2.5 to 6.5, from the standpoint of the flavor. The pH value out of the range may affect stability of the vitamin B compound. The pH value may be adjusted by adding acid or alkali according to any known method. Although the acid and alkali are unspecified as long as they are pharmaceutically acceptable, the acid may be the same one as listed below being added for the purpose of reduction of bitterness. In this case, the relevant acid works both for adjustment of the pH value and for reduction of the bitter taste.

A sweetening agent may be added to the solution of the present invention to further reduce the bitterness. The ingredient(s) and blended amount(s) thereof may be determined according to any know method. Examples of the sweetening agent include: sugar, fruit sugar, glucose or grape sugar, maltose, liquid sugar, fruit-grape-liquid sugar, grape-fruit-liquid sugar, invert-type liquid sugar, trehalose, palatinose, maltitol, , sorbitol, palatinit, erythritol, xylitol, sormatine, sucrose, and stevia extracted refined material. They may be employed alone, or two or more of them may be employed together.

Organic acid or inorganic acid may further be blended to the solution of the present invention, which will further reduce the bitterness. The organic acid may include citric acid, DL-malic acid, tartaric acid, lactic acid, glutamic acid, and aspartic acid. The inorganic acid may include hydrochloric acid and phosphoric acid. Among them, citric acid, DL-malic acid and phosphoric acid are most preferable. They may be employed alone, or two or more of them may be employed together.

Besides the components described above, those generally usable for the oral intake solutions may be blended into the solution of the present invention as desired. The ingredient(s) and blended amount(s) thereof may be determined according to any known method. Such components include: aminoethylsulfonic acid, aspartic acid, arginine, lysine, ascorbic acid, nicotinic acid amide, vitamin A or its derivative, vitamin E or its derivative, carnitine chloride, chondroitin sulfate, caffeine, crude drugs (e.g., ginseng, Eucommia ulmoides, Lurong or young deer horn, cinnamon or cassia bark, guarana or Paullinia cupana, peony or Paeonia albiflora, Jujube or zizyphi fructus, bread or ginger, glycyrrhiza or Glycyrrhiza glabra, hippocampus, cistanchis herba, morindae radix, astragali radix, angericae radix or Japanese angelica root, hoelen or Pachyma hoelen, atractylodis rhizoma, royal jelly) and other components useful for organisms; solutions such as propylene glycol; and preservatives. They may be employed alone, or two or more of them may be employed together.

The oral intake solution of the present invention may be administered in the same manner as a common solution administered orally.

### Examples

Hereinafter, the present invention will be described in more detail with reference to examples and comparative examples, although they should not be taken by way of limitation.

### Example 1

| | |
|---|---|
| fursultiamine hydrochloride | 2.5 mg |
| riboflavin | 2 mg |
| pyridoxine hydrochloride | 10 mg |
| nicotinic acid amide | 25 mg |
| sodium L-aspartate | 125 mg |
| aminoethyl sulfonate | 1000 mg |
| anhydrous caffeine | 50 mg |
| citric acid | 350 mg |
| tartaric acid | 100 mg |
| DL-malic acid | 100 mg |
| monosodium L-glutamate | 5 mg |
| purified sucrose | 9 g |
| reduced maltose starch syrup | 1.5 g |
| sodium benzoate | 60 mg |
| ethyl parahydroxybenzoate | 6 mg |
| flavoring agent | infinitesimal dose |
| electrolytic reduced water | added to 100 mL |

The ingredients above were mixed and dissolved into the water, which was filtered by a membrane filter of 0.45 µm to obtain a test solution. The pH value of the solution was from 2.9 to 3.1.

### Example 2

| | |
|---|---|
| fursultiamine hydrochloride | 5 mg |
| riboflavin | 2 mg |
| pyridoxine hydrochloride | 10 mg |
| nicotinic acid amide | 25 mg |
| sodium L-aspartate | 125 mg |
| aminoethyl sulfonate | 1500 mg |
| anhydrous caffeine | 50 mg |
| citric acid | 350 mg |
| tartaric acid | 100 mg |
| DL-malic acid | 100 mg |
| monosodium L-glutamate | 5 mg |
| sodium chloride | 40 mg |
| purified sucrose | 8 g |
| fruit sugar | 2 g |
| reduced maltose starch syrup | 1 g |
| sodium benzoate | 60 mg |
| ethyl parahydroxybenzoate | 6 mg |
| flavoring agent | infinitesimal dose |
| electrolytic reduced water | added to 100 mL |

The ingredients above were mixed and dissolved into the water, which was filtered by a membrane filter of 0.45 µm to obtain a test solution. The pH value of the solution was from 2.9 to 3.1.

### Example 3

| | |
|---|---|
| fursultiamine hydrochloride | 5 mg |
| riboflavin | 2 mg |
| pyridoxine hydrochloride | 10 mg |
| nicotinic acid amide | 25 mg |
| sodium L-aspartate | 125 mg |
| aminoethyl sulfonate | 1500 mg |
| anhydrous caffeine | 50 mg |
| citric acid | 350 mg |
| tartaric acid | 100 mg |
| DL-malic acid | 100 mg |
| monosodium L-glutamate | 5 mg |
| sodium chloride | 40 mg |
| purified sucrose | 8 g |
| fruit sugar | 2 g |
| powder of reduced maltose starch syrup | 1 g |
| sodium benzoate | 60 mg |
| ethyl parahydroxybenzoate | 6 mg |
| flavoring agent | infinitesimal dose |
| electrolytic reduced water | added to 100 mL |

The ingredients above were mixed and dissolved into the water, which was filtered by a membrane filter of 0.45 µm to obtain a test solution. The pH value of the solution was from 2.9 to 3.1.

### Example 4

| | |
|---|---|
| fursultiamine hydrochloride | 5 mg |
| riboflavin | 2 mg |
| pyridoxine hydrochloride | 10 mg |
| nicotinic acid amide | 25 mg |
| sodium L-aspartate | 125 mg |
| anhydrous caffeine | 50 mg |
| citric acid | 150 mg |
| tartaric acid | 100 mg |
| DL-malic acid | 50 mg |
| purified sucrose | 9 g |
| sodium benzoate | 30 mg |
| ethyl parahydroxybenzoate | 2.5 mg |
| flavoring agent | infinitesimal dose |
| electrolytic reduced water | added to 50 mL |

The ingredients above were mixed and dissolved into the water, which was filtered by a membrane filter of 0.45 µm to obtain a test solution. The pH value of the solution was from 3.0 to 3.2.

### Example 5

| | |
|---|---|
| fursultiamine hydrochloride | 5 mg |
| sodium riboflavin phosphate | 2 mg |
| pyridoxine hydrochloride | 10 mg |
| nicotinic acid amide | 25 mg |
| sodium L-aspartate | 125 mg |
| anhydrous caffeine | 50 mg |
| citric acid | 150 mg |
| tartaric acid | 100 mg |
| DL-malic acid | 50 mg |
| purified sucrose | 9 g |
| sodium benzoate | 30 mg |
| butyl parahydroxybenzoate | 2.5 mg |
| flavoring agent | infinitesimal dose |
| electrolytic reduced water | added to 50 mL |

The ingredients above were mixed and dissolved into the water, which was filtered by a membrane filter of 0.45 µm to obtain a test solution. The pH value of the solution was from 3.0 to 3.2.

### Example 6

| | |
|---|---|
| fursultiamine hydrochloride | 10 mg |
| sodium riboflavin phosphate | 5 mg |
| pyridoxine hydrochloride | 10 mg |
| nicotinic acid amide | 30 mg |
| sodium L-aspartate | 125 mg |
| aminoethyl sulfonate | 1000 mg |
| carnitine chloride | 100 mg |
| sodium chondroitin sulfate | 120 mg |
| anhydrous caffeine | 50 mg |
| citric acid | 330 mg |
| sodium citrate | 30 mg |
| tartaric acid | 50 mg |
| lactic acid | 0.1 mL |
| purified sucrose | 7 g |
| honey | 3.5 g |
| sodium benzoate | 35 mg |
| ethyl parahydroxybenzoate | 2.5 mg |
| flavoring agent | infinitesimal dose |
| electrolytic reduced water | added to 50 mL |

The ingredients above were mixed and dissolved into the water, which was filtered by a membrane filter of 0.45 µm to obtain a test solution. The pH value of the solution was from 2.9 to 3.1.

### Comparative example 1

| | |
|---|---|
| fursultiamine hydrochloride | 2.5 mg |
| riboflavin | 2 mg |
| pyridoxine hydrochloride | 10 mg |
| nicotinic acid amide | 25 mg |
| sodium L-aspartate | 125 mg |
| aminoethyl sulfonate | 1000 mg |
| anhydrous caffeine | 50 mg |
| citric acid | 350 mg |
| tartaric acid | 100 mg |
| DL-malic acid | 100 mg |
| monosodium L-glutamate | 5 mg |
| purified sucrose | 9 g |
| reduced maltose starch syrup | 1.5 g |
| sodium benzoate | 60 mg |
| ethyl parahydroxybenzoate | 6 mg |
| flavoring agent | infinitesimal dose |
| purified water | added to 100 mL |

The ingredients above were mixed and dissolved into the water, which was filtered by a membrane filter of 0.45 µm to obtain a test solution.

The pH value of the solution was from 2.9 to 3.1.

### Comparative example 2

| | |
|---|---|
| fursultiamine hydrochloride | 5 mg |
| riboflavin | 2 mg |
| pyridoxine hydrochloride | 10 mg |
| nicotinic acid amide | 25 mg |
| sodium L-aspartate | 125 mg |
| aminoethyl sulfonate | 1500 mg |
| anhydrous caffeine | 50 mg |
| citric acid | 350 mg |
| tartaric acid | 100 mg |
| DL-malic acid | 100 mg |
| monosodium L-glutamate | 5 mg |
| sodium chloride | 40 mg |
| purified sucrose | 8 g |
| fruit sugar | 2 g |
| reduced maltose starch syrup | 1 g |
| sodium benzoate | 60 mg |
| ethyl parahydroxybenzoate | 6 mg |
| flavoring agent | infinitesimal dose |
| purified water | added to 100 mL |

The ingredients above were mixed and dissolved into the water, which was filtered by a membrane filter of 0.45 µm to obtain a test solution. The pH value of the solution was from 2.9 to 3.1.

### Comparative example 3

| | |
|---|---|
| fursultiamine hydrochloride | 5 mg |
| riboflavin | 2 mg |
| pyridoxine hydrochloride | 10 mg |
| nicotinic acid amide | 25 mg |
| sodium L-aspartate | 125 mg |
| aminoethyl sulfonate | 1500 mg |
| anhydrous caffeine | 50 mg |
| citric acid | 350 mg |
| tartaric acid | 100 mg |
| DL-malic acid | 100 mg |
| monosodium L-glutamate | 5 mg |
| sodium chloride | 40 mg |
| purified sucrose | 8 g |
| fruit sugar | 2 g |
| powder of reduced maltose starch syrup | 1 g |
| sodium benzoate | 60 mg |
| ethyl parahydroxybenzoate | 6 mg |
| flavoring agent | infinitesimal dose |
| purified water | added to 100 mL |

The ingredients above were mixed and dissolved into the water, which was filtered by a membrane filter of 0.45 µm to obtain a test solution. The pH value of the solution was from 2.9 to 3.1.

### Comparative example 4

| | |
|---|---|
| fursultiamine hydrochloride | 5 mg |
| riboflavin | 2 mg |
| pyridoxine hydrochloride | 10 mg |
| nicotinic acid amide | 25 mg |
| sodium L-aspartate | 125 mg |
| anhydrous caffeine | 50 mg |
| citric acid | 150 mg |
| tartaric acid | 100 mg |
| DL-malic acid | 50 mg |
| purified sucrose | 9 g |
| sodium benzoate | 30 mg |
| ethyl parahydroxybenzoate | 2.5 mg |
| flavoring agent | infinitesimal dose |
| purified water | added to 50 mL |

The ingredients above were mixed and dissolved into the water, which was filtered by a membrane filter of 0.45 µm to obtain a test solution. The pH value of the solution was from 3.0 to 3.2.

### Comparative example 5

| | |
|---|---|
| fursultiamine hydrochloride | 5 mg |
| sodium riboflavin phosphate | 2 mg |
| pyridoxine hydrochloride | 10 mg |
| nicotinic acid amide | 25 mg |
| sodium L-aspartate | 125 mg |
| anhydrous caffeine | 50 mg |
| citric acid | 150 mg |
| tartaric acid | 100 mg |
| DL-malic acid | 50 mg |
| purified sucrose | 9 g |
| sodium benzoate | 30 mg |
| butyl parahydroxybenzoate | 2.5 mg |
| flavoring agent | infinitesimal dose |
| purified water | added to 50 mL |

The ingredients above were mixed and dissolved into the water, which was filtered by a membrane filter of 0.45 µm to obtain a test solution. The pH value of the solution was from 3.0 to 3.2.

### Comparative example 6

| | |
|---|---|
| fursultiamine hydrochloride | 10 mg |
| sodium riboflavin phosphate | 5 mg |
| pyridoxine hydrochloride | 10 mg |
| nicotinic acid amide | 30 mg |
| sodium L-aspartate | 125 mg |
| aminoethyl sulfonate | 1000 mg |
| carnitine chloride | 100 mg |
| sodium chondroitin sulfate | 120 mg |
| anhydrous caffeine | 50 mg |
| citric acid | 330 mg |
| sodium citrate | 30 mg |
| tartaric acid | 50 mg |
| lactic acid | 0.1 mL |
| purified sucrose | 7 g |
| honey | 3.5 g |
| sodium benzoate | 35 mg |
| ethyl parahydroxybenzoate | 2.5 mg |
| flavoring agent | infinitesimal dose |
| purified water | added to 50 mL |

The ingredients above were mixed and dissolved into the water, which was filtered by a membrane filter of 0.45 µm to obtain a test solution. The pH value of the solution was from 2.9 to 3.1.

### Test Example 1

### Sensory evaluation regarding bitterness

Ten healthy adults were selected to conduct a sensory test on bitterness. Each specimen was tested and evaluated in four levels of "bitter", "slightly bitter", "hardly bitter" and "not bitter". The specimens were tested at random by each test subject. Table 1 shows the results.

**Table 1:**

| Result of sensory test on bitterness (unit: person) | | | | |
|---|---|---|---|---|
| Specimen | bitter | slightly bitter | hardly bitter | not bitter |
| Example 1 | 0 | 0 | 0 | 10 |
| Example 2 | 0 | 0 | 0 | 10 |
| Example 3 | 0 | 0 | 0 | 10 |
| Example 4 | 0 | 0 | 1 | 9 |
| Example 5 | 0 | 0 | 2 | 8 |
| Example 6 | 0 | 1 | 7 | 2 |
| Comparative Example 1 | 0 | 1 | 9 | 0 |
| Comparative Example 2 | 0 | 4 | 6 | 0 |
| Comparative Example 3 | 0 | 2 | 8 | 0 |
| Comparative Example 4 | 0 | 4 | 6 | 0 |
| Comparative Example 5 | 0 | 3 | 7 | 0 |
| Comparative Example 6 | 1 | 4 | 5 | 0 |

As seen from Table 1, while almost all the Comparative examples were evaluated as "slightly bitter" or "hardly bitter", most of the Examples of the present invention were evaluated as "hardly bitter" or "not bitter".

As such, according to the present invention, an oral intake solution reduced in bitterness of vitamin B compounds and hence suitable for oral administration is provided.

The electrolytic reduced water used in Examples 1 to 6 was prepared by subjecting purified water containing 0.01 % (by weight) sodium chloride to electrolysis using a Nihon Trim TI-8000 model electrolyzer at an operating current of 5 ampere. The resulting reduced water has an oxidation potential of -729 mV and contains 1030 ppb dissolved hydrogen.

## Claims

1. A method for reducing bitterness **characterized in that** a vitamin B compound as a water-soluble component presenting a bitter taste is dissolved into reduced water, having an oxidation potential of less than 0mV.

2. The method according to claim 1, wherein the reduced water is electrolytic reduced water.

3. An oral intake solution including a vitamin B compound at a water-soluble component presenting a bitter taste and reduced water, having an oxidation potential of less than 0mV.

4. The oral intake solution according to claim 3, wherein the reduced water is electrolytic reduced water.

5. The oral intake solution according to claim 4, having a pH value in a range from 2 to 7.

6. A method for producing the oral intake solution according to claim 3, **characterized in that** the vitamin B compound is dissolved into the reduced water.

## Patentansprüche

1. Verfahren zur Verringerung von Bitterkeit, **dadurch gekennzeichnet, dass** eine Vitamin B-Verbindung als wasserlösliche Komponente, die einen bitteren Geschmack aufweist, in reduziertem Wasser, das ein Oxidationspotential von kleiner als 0 mV hat, gelöst wird.

2. Verfahren nach Anspruch 1, wobei das reduzierte Wasser elektrolytisch reduziertes Wasser ist.

3. Lösung zur oralen Einnahme, die eine Vitamin B-Verbindung als wasserlösliche Komponente, die einen bitteren Geschmack aufweist, und reduziertes Wasser, das ein Oxidationspotential von kleiner als 0 mV hat, enthält,

4. Lösung zur oralen Einnahme nach Anspruch 3, wobei das reduzierte Wasser ein elektrolytisch reduziertes Wasser ist.

5. Lösung zur oralen Einnahme nach Anspruch 4, die einen pH-Wert im Bereich von 2 bis 7 hat.

6. Verfahren zur Herstellung einer Lösung zur oralen Einnahme nach Anspruch 3, **dadurch gekennzeichnet, dass** die Vitamin B-Verbindung in dem reduzierten Wasser gelöst wird.

## Revendications

1. Procédé pour réduire l'amertume, **caractérisé en ce qu'**un composé vitamine B en tant que composant soluble dans l'eau présentant un goût amer est dissous dans de l'eau réduite ayant un potentiel d'oxydation inférieur à 0 mV.

2. Procédé selon la revendication 1, dans lequel l'eau réduite est de l'eau réduite par électrolyse.

3. Solution pour prise orale incluant un composé vitamine B en tant que composant soluble dans l'eau présentant un goût amer et de l'eau réduite, ayant un potentiel d'oxydation inférieur à 0 mV.

4. Solution pour prise orale selon la revendication 3, dans laquelle l'eau réduite est de l'eau réduite par électrolyse.

5. Solution pour prise orale selon la revendication 4, ayant une valeur de pH dans la zone de 2 à 7.

6. Procédé pour produire la solution pour prise orale selon la revendication 3, **caractérisé en ce que** le composé vitamine B est dissous dans l'eau réduite.
